# EUROPEAN PATENT SPECIFICATION

(11) **EP 4 268 790 B1**
(45) Date of publication and mention of the grant of the patent: **06.11.2024**
(21) Application number: 23020120.4
(22) Date of filing: 13.03.2023
(51) Int. Cl.: A61J 7/00, A61J 7/04

(54) **AUTOMATED DISCRETE PARTICLE DISPENSER AND SYSTEM**
AUTOMATISIERTER SPENDER FÜR DISKRETE PARTIKEL UND SYSTEM
DISTRIBUTEUR AUTOMATIQUE DE PARTICULES DISCRETES ET SYSTEME ASSOCIE

(30) Priority: 29.04.2022 AR P220101130
(43) Date of publication of application: 01.11.2023
(73) Proprietor: Simaro, Eduardo Marcos Ezequiel, 56128 Pisa (PI) (IT)
(72) Inventor: Simaro, Eduardo Marcos Ezequiel, 56128 Pisa (PI) (IT)

(56) References cited:
- US-A1- 2009 294 521
- US-A1- 2022 047 461
- US-B2- 10 555 873

## Description

### TECHNICAL FIELD

The present invention belongs to the field of devices for providing scheduled delivery of discrete particles, in particular, without this being a limitation to the scope of the invention, to the scheduled delivery of medications, such as capsules and pills of medicinal specialties.

### BACKGROUND ART

The scheduled administration of medications in the form of pills, capsules, tablets or the like, especially for patients with a permanent need to receive said medications, is a recurrent problem that becomes more significant day by day.

It is a proven fact that most treatment failures are caused by lack of adherence and compliance with medicinal therapeutics, either due to oversight, or to difficulty of access.

This problem is aggravated in bedridden patients, either old age or chronic patients residing in nursing homes or similar facilities.

The pandemic has brought to light this problem that affects many elderly people or isolated chronic patients.

In addition to patients hospitalized in care units, there is a significant population in need of permanent medical treatment. The growth of this fraction of the elderly population is due to longer life expectancy, which has led to a significantly higher proportion of elderly patients in need of permanent care.

Within the medical care scheme, administration of medications according to the dosage prescribed by a physician or doctor and at the correct intake time, implies a more than relevant effort in logistics, organization and control, with the resulting increase in nursing hours, and a high increase in the costs of care for this population group.

To overcome these drawbacks, various devices have been developed, starting with the traditional divided pill boxes, usually for seven days a week, containing daily intake medications in each division. This solution assumes that the patient is capable of remembering the times at which he or she must take the medication, and does not solve the problem of the nursing hours devoted to each patient.

Consequently, various devices have been designed for the automated delivery of medication, and particularly, that are capable of producing a signal, for example an audio signal, when dispensing medication at a predetermined time.

As specific examples of such devices, there are carousels dispensers or disk pill boxes, with partitions that define radial separations, each containing one or more doses according to medical prescription. These discs take up significant space and do not allow duplication of the dose load, unless several discs are piled one on top of the other, complicating the problem of occupied space.

In particular, it is worth mentioning the existence of document US2022047461A1 which covers a pill dispenser that has a core with outward-facing compartments arranged in rows and layers about a central axis. A first sleeve overlaying the core has an elongated slot extending parallel to the central axis, and is rotatable relative to the first sleeve to align the slot with one of the rows of the compartments. A second sleeve, coaxial with the first sleeve, has a pattern of staggered openings positioned in an axial direction to align with a corresponding one of the layers. The second sleeve is rotatable relative to the first sleeve to bring each of the staggered openings into alignment with the elongated slot, thereby providing access to one compartment of the core. Preferably, the core includes a number of axially removable cartridges, each including a row of compartments, that can be inserted while the compartments contain pills. This construction is complicated, employing a plurality of parts for loading the medications, and also does not allow joining several units so that they work in a staggered and scheduled manner, at the same time that it is not possible to remove a single unit, load the medication and replace it by means of a simple manual operation.

### SUMMARY

Said and other inconvenient of the state of the art are now solved by the first object of the present invention and, in particular, by a particle dispenser comprising a casing; a first body accommodated in said casing and comprising a plurality of first housings capable of containing a number of discrete particles; a second body accommodated in said casing and comprising a plurality of second housings. The first body is configured to move relative to said casing between a first operating position in which each housing of the plurality of first housings communicates with a corresponding housing of the second plurality of second housings through a window and a second operating position in which the first housings are isolated from the second housings. The second body is configured to selectively move relative to said casing between an initial position to a final position passing through intermediate positions, wherein in each of said positions at least one second housing communicates with a release mouth of the casing. Said positions can include or exclude the initial position. The dispenser device so conceived allows to dispense a certain number of particles, namely those in the second body, and to refill the second body with the particles of the first body, once the first group of particles of the first body are finished. In this way, the autonomy of the device is increased and the patient can receive autonomously medications for a longer time. Moreover, the device can continue to dispense drugs from the second body, while the first body is refilled with new drugs. Furthermore, this solution is reliable and simple to build and operate, and is suitable for a scheduled dispensing of discrete particles like pills, tablets and capsules, even with their blisters if properly cut out for being easily accommodate in the housings. Being the housings sufficiently large, a weakness of most dispensers available on the market is solved, because medicament do not need to be unpacked and with their original packaging preserve their pharmacological efficacy longer.

Advantageously, the casing can comprise a first casing shaped for accommodating the first body and a second casing shaped for accommodating the second body. In this way, the particles in the first body remain isolated from the particles in the second body.

Preferably, the first casing can define a hollow cylindrical bore having a longitudinal axis and the first body can be cylindrically shaped and arranged coaxial to the axis of the hollow cylindrical bore of said first casing. More preferably said axis can be arranged horizontal or substantially horizontal. In this way, the first body can rotate into the first casing in a simple manner.

In particular, the first body can comprise a cylindrical sidewall that is partially opened along a longitudinal direction and a plurality of partitions arranged, inside this sidewall, so as to define said plurality of first housings. Preferably said partitions can be perpendicular to the longitudinal axis of the first body. The first body acts as a multi-housings container that is open on one side and closed on the opposite side. The first body so defined can overturn rotating about its axis, bringing down the particles contained in its first housings.

Advantageously, the hollow cylindrical bore of the first casing can be opened at one or both ends and the first body can be selectively removable from the first casing through one of its ends for refilling it. In this way, the first body can be easily extracted from the first casing and easily refilled with other particles or pills, even if the rest of the particle dispensers continues to work.

Preferably, the second body can be prismatically coupled to the second casing, so that the second body longitudinally moves along a direction that is substantially parallel to the longitudinal axis of the first casing. This kind of movement allows a discrete dispensing of particles from the first body.

Advantageously, the second body can comprise a sidewall having top and bottom apertures and a plurality of partitions arranged so as to define said plurality of second housings. This inner architecture of the second body allows to define second housings that are laterally closed and opened above and below.

In particular, the first body can be moved by a first motor and the second body can be moved by a second motor. In this manner, the movement of the first body is independent from the movement of the second body. Preferably the first motor can be reversibly coupled to the first body through a quick coupling. This feature allows a simple extraction of the first body from the device for refilling it with new particles.

Preferably, the particle dispenser can comprise a control unit which is configured to control the second motor so as to move forward the second body from the initial position to each of the intermediate positions in a step by step manner until the final position is reached and backward from the final position to the initial position. In this manner, the control unit intermittently commands the movement of the second body and, according to a loop, the second body is moved forward from the initial position toward the final position and backward from the final position to the initial position once again.

In particular, the control unit can move forward the second body from a position to the subsequent position according to a predetermined time interval or according to a command that is provided to the control unit, for example through a human-machine interface or a feed button. In this manner, the second body advances, step by step, after a specific interval or if the user decides to command it.

Specifically, the control unit can be configured to move the first body in the first operating position only when the second body is positioned in said initial position. This feature allows to withdraw the content of the first body in the second body only when the first and second housings are aligned.

A second object of the present invention is that of providing a dispensing system comprising a plurality of particle dispensers according to one or more of preceding claims; convey means configured to collect and convey the particles expelled from the particle dispensers to a collecting tray. The system according to the present invention allows to organize the intake of various medications for multi-medicated chronic patients, using the dynamic combination of medications and their scheduled administration in time and quantity. In this way, elderly people living alone can be supervised by their family members.

Preferably, the convey means can comprise a belt conveyor actuated by a third motor, which ends in the collecting tray. Alternatively, the convey means can comprise an inclined/vertical tube, which ends in the collecting tray. In this manner, each time that at least a particle is released by a dispenser device, the particle/s is/are conveyed to the collecting tray. In the first case, each particle is transported to the collecting tray by the belt conveyor, while in the second case each particle arrives to the collecting tray by gravity.

Advantageously, the dispensing system can comprise a central control unit which substitutes or coordinates the control units of the particle dispensers and a human-machine interface and/or a wireless communication system configured to communicate with a mobile device of a user. In this manner, the central control unit can control all the dispenser devices, which for examples contains different types of particles. Optionally, the human-machine interface allows to program the central control unit. The same result can be also achieved via a smartphone or tablet of a user and a wireless interface that creates a communication between the smartphone/tablet and the central control unit. In this manner, the dispensing of medications can be planned and it proceeds automatically. The system so conceived is capable of scheduling the delivery of medication at certain intervals and predefined times. The system is thus able to delivery medications/drugs for an extended period of time, for example, the whole week or month.

Preferably, the dispensing system can comprise an indicator light configured to turn on when the first body of a particle dispenser is empty, in order to inform a user that the specific particle dispenser needs to be refilled. The dispensing system can also comprise a warning configured to switch-on when a particle is contained in the collecting tray for informing a user that a medication/particle needs to be assumed.

Finally, the invention relates to an automated discrete particle dispenser comprising at least one body with a plurality of compartments, each capable of containing a number of discrete particles to be delivered in a pre-scheduled manner, characterized by having a first cylindrical casing disposed with its axis in a substantially horizontal position; said first casing defining a hollow cylindrical bore open at both ends, with a lower elongated lateral opening defining a first downward dispensing window; wherein said first casing fully houses a first cylindrical body coaxial to the axis of the first casing, formed by a half-round that defines a closed base, open on its portion of the side opposite to said closed base, comprising a plurality of partitions substantially perpendicular to the longitudinal axis of the first body and arranged between the ends thereof that define a plurality of first housing cells, said first body being selectively removable from the first casing through one of its ends; connected to said first casing and having a longitudinal axis parallel to its axis, a second casing is connected comprising a bottom and on its opposite face a second window arranged parallel and matching the first window, said second casing having a second body with a cross-section complementary to that of the second casing, comprising side walls and a plurality of partitions that define a succession of second housings, matching vertically with the first housings, each of said housings of the second body being open passages without top or bottom walls; the first body rotates on its axis between two positions, the first being with its bottom covering the first window and in the second position the first window is connected to the second window of the second body, allowing free fall and entry into each second housing of the discrete particles originally contained in the housings of the first body; said first body being rotated over its axis by a first motor means, having the end of the first body coupled to said first motor means by a quick coupling, selectively detachable, this first body being selectively removable from the first housing from the opposite end of the one with the first motor means; the second body moves selectively along the second housing dragging the particles held therein, until reaching the mouth of a release hopper located at the bottom of the second casing, this second body being moved axially along said second casing by a second motor means acting on complementary means integral with said second body.

These and other advantages will be better understood thanks to the following description of different embodiments of said invention given as non-limitative examples thereof, making reference to the annexed drawings.

### DRAWINGS DESCRIPTION

In the drawings:
Figure 1 is a schematic exploded view of a particle dispenser according to the present invention;
Figure 2 is a schematic side view of the first body in the receiving position of discrete particles;
Figure 3 is a schematic top view of the first body;
Figure 4 shows section A-A of first body of fig. 3;
Figure 5 shows section B-B of first body of fig. 3;
Figure 6 is the first body of Fig. 3 viewed from the removable end thereof;
Figure 7 is the first body of Fig. 3 viewed from the end that is attached to the first motor;
Figure 8 is a schematic side view of the second body;
Figure 9 is a schematic top view of the second body;
Figure 10 is a schematic and partial perspective view of the connection system between first body and the first motor;
Figure 11 is a schematic cross-section view of the particle dispenser in which the first body is in a second operating position and the second body is in an initial position;
Figure 12 is a schematic cross-section view of the particle dispenser in which the first body is in a first operating position and the second body is in an initial position;
Figure 13 is a schematic cross-section view of the particle dispenser in which the first body is in a first operating position and the second body is in an intermediate position;
Figure 14 is a schematic cross-section view of the particle dispenser in which the first body is in a first operating position and the second body is in a final position;
Figure 15 shows a front view of a multiple construction in which a number of particle dispensers according to the present invention are mounted together to form a dispensing system;
Figure 16 shows a side view of the dispensing system of Fig. 15;
Figure 17 shows a top view of the dispensing system of Fig. 15;
Fig. 18 is a perspective view of a dispensing system according to the present invention in which an outer casing is partially transparent and from which a particle dispenser has been removed;
Fig. 19 is a perspective view of a dispensing system according to the present invention in which an outer casing is partially transparent;
Fig. 20 is a perspective view of a dispensing system of Fig. 19 without outer casing;
Fig. 21 is another perspective view of a dispensing system of Fig. 19 without outer casing;
Fig. 22 is a perspective view of a particle dispenser according to an embodiment of the present invention;
Fig. 23 is another perspective view of the particle dispenser of Fig. 22;
Fig. 24 is a perspective exploded view of the particle dispenser of Fig. 22;
Fig. 25 is another perspective exploded view of the particle dispenser of Fig. 22;

### DETAILED DESCRIPTION

The following description of one or more embodiments of the invention is referred to the annexed drawings. The same reference numbers indicate equal or similar parts. The object of the protection is defined by the annexed claims. Technical details, structures or characteristics of the solutions here-below described can be combined with each other in any suitable way.

In order to exemplify the preferred embodiment of the present invention, the following drawings illustrating it are attached hereto, which, with the support of the description provided below, should be interpreted as one of the many possible embodiments of the invention. Therefore, it is not appropriate to assign any limiting value to it, including possible means equivalent to those shown within the scope of protection of the invention, as defined by the claims. In addition, in these figures, the same references identify the same and/or equivalent means.

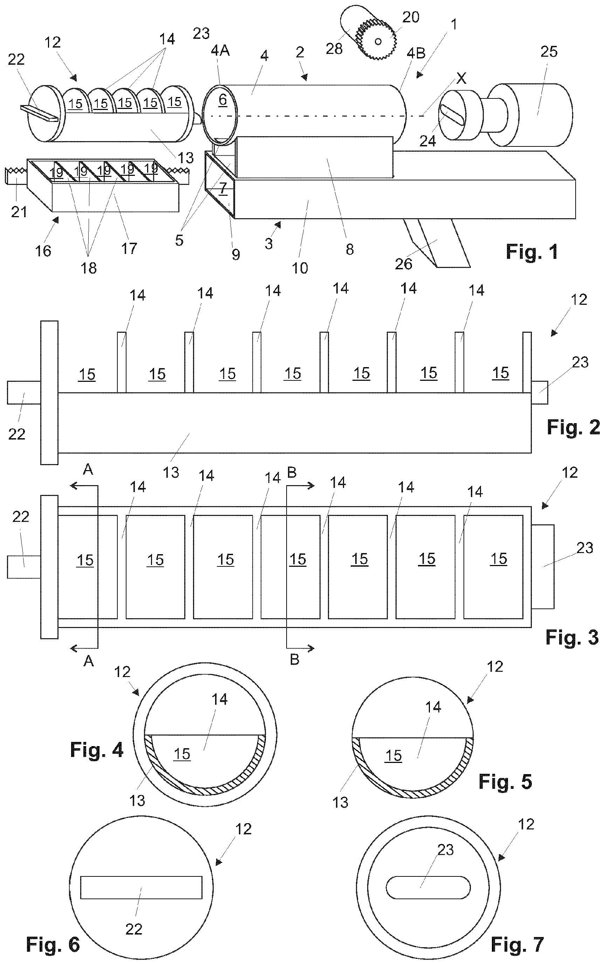

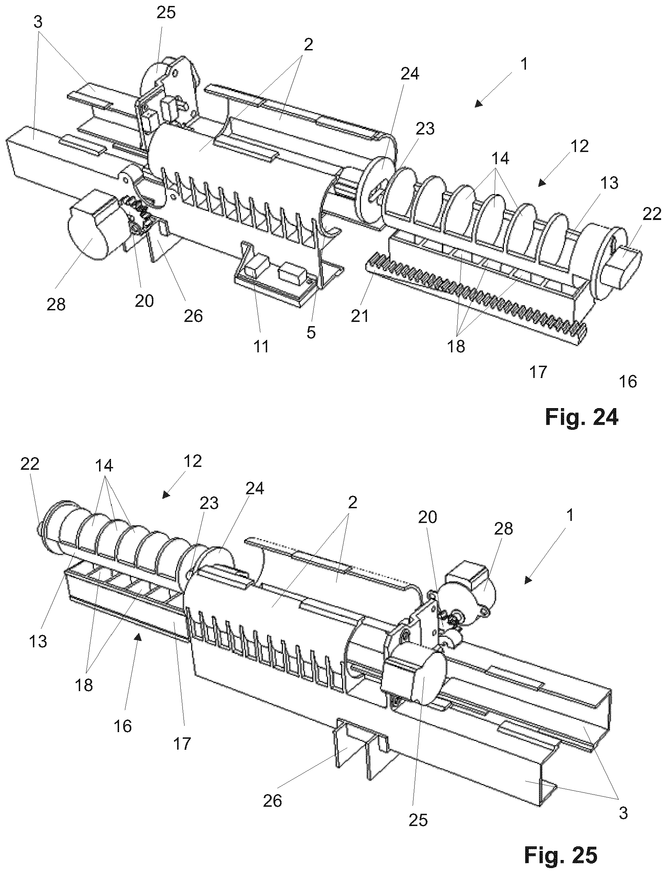

Figs. 1,24,25 depict exploded views of the device 1 of the invention with its main component parts. The discrete particle dispenser 1 comprises a casing having a first casing 2 and a second casing 3.

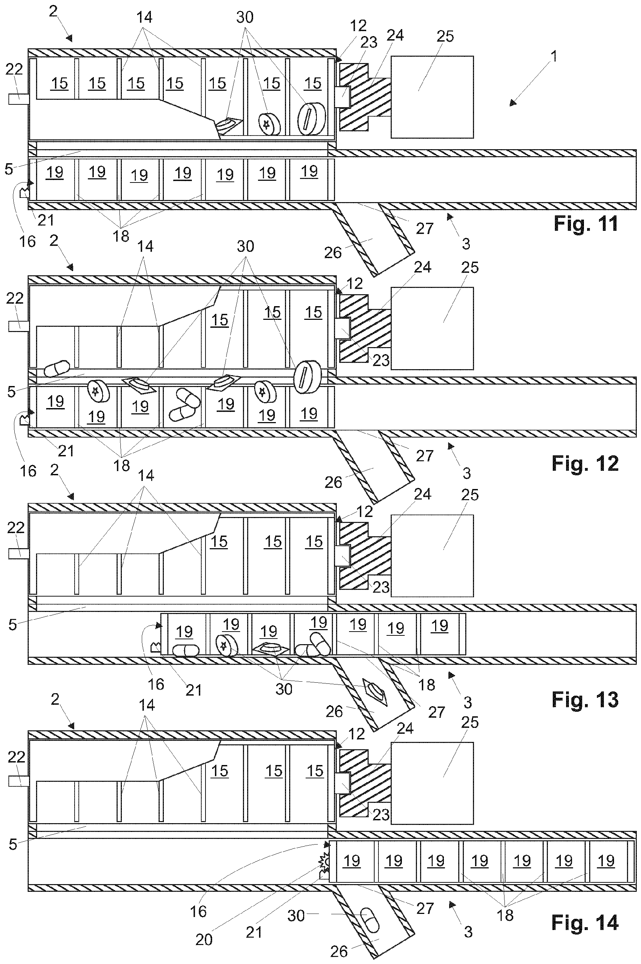

The first casing 2 has a cylindrical body 4 and comprises an inner cylindrical bore 6 with a longitudinal axis X that is substantially horizontal, also shown in Fig. 14. Alternatively, the axis X can be inclined. The bore 6 of the first casing 2 is hollow and preferably has a uniform cross-section that is open at its respective ends 4A, 4B.

The second casing 3 is solidary with the first casing 2.

The first casing 2 and the second casing 3 communicate each other through a window 5. The window 5 opens downwards from the first casing 2 towards the second casing 3, as shown in Fig.1.

This second casing 3 is shaped so as to define a hollow bore 7, which has an elongated shape and a uniform cross section. Said second casing 3 is located below the first casing 2, preferably vertically below the first casing 2. The longitudinal axis of the second casing 3 is parallel to the axis X of the first casing 2. Consequently, the second casing 3 is parallel to the first casing 2.

Preferably, the second casing 3 is attached to the first casing 2 by means of a support 8, as shown in Figs. 1, 24, 25.

Conveniently, this second casing 3 has a quadrangular cross-section made up of a flat bottom 9 and sidewall 10 perpendicular to the bottom 9. The vault of said second casing 3 is opened and comprises the window 5. Part of the window 5 is also arranged on the body 4 of the first casing 2 in order to create a passage between the first casing 2 and the second casing 3. The window 5 can also be an channel between the first and second casings 2,3.

Inside the first casing 2 a first body 12 is fully housed. The outer shape of the first body 12 is substantially complementary to the inner shape of the first casing 2. Consequently, the outer surface of the first body 12 is cylindrical. Said first body 12 is inserted in the first casing 2 through the first end 4A. Once it's inserted, the first body 12 is coaxial to the first casing 2 and consequently the longitudinal axis of the first body 12 is coaxial to the axis X of the first casing 2. In this manner, the first body 12 can rotate about the axis X inside the first casing 2.

The first body 12 is actuated by a first motor 25, as shown in Figs. 1, 24 and 25. Preferably the first motor 25 is an electric motor.

As shown in Figs. 6 and 7, the first body 12 comprises a connector 23, arranged at the opposite end with respect to a handle 22. The handle 22 is used for grasping the first body 12 in order to extract it from the first casing 2. The connector 23 is shaped so as to mateably couple to a quick coupling 24. The quick coupling 24 is in turn attached to a shaft of the first motor 25. When the first body 12 is inserted in the first casing 2, the connector 23 cantilevers from the first casing 2, as shown in Figs. 11-14. The connector 23 enters in the quick coupling 24 and consequently the rotary movement of the first motor 25 is transmitted to the first body 12, which rotates inside the first casing 2. This projection-recess, like a male-female coupling, defines a selectively detachable plug-in connection, that allows a removal of the first body 12 from the first casing 2.

As shown in Figs. 2, 3, 4, 5, the first body 12 comprises a semi-cylindrical wall 13 in the shape of a half-round that defines a hollow bore, leaving its opposite cylindrical side portion open, that is, without any wall and open, giving access to the hollow bore of the first body 12. Inside this hollow bore there is a number of partitions 14, substantially perpendicular to the longitudinal axis of the first body 12, that are arranged between the opposite ends of the first body 12. Between a pair of consecutive partitions 14 a first housing 15 is formed. The bottom of each housing 15 is thus realized by a portion of said semi-cylindrical sidewall 13, as shown in Figs. 4 and 5. Each housing 15 is dimensioned to contain one or more particles 30, as shown in Figs. 11-14.

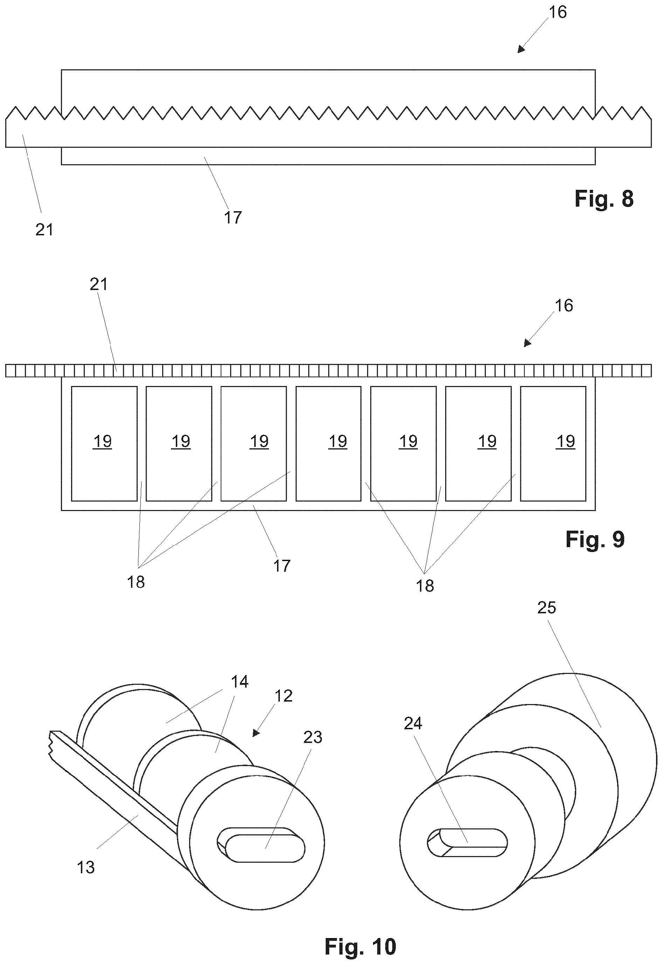

As shown in Figs. 8 and 9, the second casing 3 houses a second body 16. The outer shape of the second body 16 is substantially complementary to the inner shape of the second casing 3. Preferably, the second casing 3 has a quadrangular cross-section, but nothing prevents said cross-sections from being polyhedral, or elliptical or even circular. As already mentioned, the bore 7 of the second casing 3 is hollow to form a rectilinear passage along which said second body 16 moves axially. Said second body 16 is made up of a sidewall 17 and a plurality of partitions 18, arranged inside said sidewall 17. Between a pair of consecutive partitions 18 a second housing 19 is formed. The lateral sides of each second housing 19 are realized through portions of the sidewall 17.

The partitions 14 of the first body 12 and the partitions 18 of the second body 16 are arranged so that each first housing 15 vertically matches a corresponding second housing 19. Therefore, if the first body 12 has "n" first housings 15, the second body 16 has the same number of "n" second housings 19. Preferably the distance between immediately consecutive partitions 15 of the first body 12 is the same for the consecutive partitions 18 of the second body 16, so that in a preferred embodiment of the invention the first housings 15 have substantially the same size as the second housings 19, and ideally a similar volume.

A particular feature of the present invention is given by the fact that the second body 16 has its second housings 19 without bottom or top walls or vaults, so as to form vertical passages, as shown in Figs. 8 and 9. Consequently, the particles or medications 30 that fall into a first housing 19 from above, thus from the first body 12, remain into the first housing 19 until the bottom 9 of the second casing 3 lies below this first housing 19. Vice versa, when this first housing 19 lies in correspondence of a release opening 27 of the second casing 3, the particle/medication 30 falls into a release mouth 26, as shown in Figs. 12-14.

The second body 16 is axially movable along the second casing 3 in operating positions that are detailed below. This movement of the second body 16 is carried out by a second motor 28 which, by way of non-limiting example, acts on a toothed wheel 20 which engages with a rack 21 joined to the second body 16, as shown in Figs. 8 and 9, realizing said rectilinear and axial movement of said second body 16 into the second casing 3, as shown in Figs. 11-14, 22 and 24.

Figs. 1, 11-14 and 22-25 show a release mouth 26 attached to the second casing 3, in correspondence of a release opening 27 of the bottom 9 of the second casing 3. Through the release mouth 26 the particles 30 contained in the device 1 can exit.

The operation of the present invention is explained with reference to Figs. 11-14.

The first body 12 is initially removed through end 4A of the first casing 2 by grabbing the handle 22 and pulling it. Once that the first body 12 is extracted from the first casing 2, it can be loaded (not shown step) with particles or medications 30. During loaded, the semi-cylindrical sidewall 13 of the first body 12 faces down. In Fig. 11 is shown the first body 12 re-inserted in the first casing 2 once the loading step is ended. The particles 30 that can be used with this device 1 can be unpackaged medications, like pills, or medications with their blisters, as shown in Figs. 11-14.

Once said first body 12 has been loaded, it is placed inside the first casing 2 and the connector 23 is coupled to the quick coupling 24 of the first motor 25.

The second body 16 with its rack 21 is placed into the second casing 3 so that each second housing 19 is vertically aligned with each first housing 15 of the first body 12. The partitions 14,18 of the first and second bodies 12,16 are consequently aligned. This arrangement corresponds to the initial position of the second body 16 shown in Fig. 11.

The first body 12 is initially arranged so that the semi-cylindrical sidewall 13 faces downwards. In this manner, the window 5 is closed by the semi-cylindrical sidewall 13 and the particles 30 remain in the first body 12, as shown in Fig. 11.

The first body 12 is then rotated about its axis X of 180 degrees by means of the first motor 25, so that the semi-cylindrical sidewall 13 is facing up, and consequently the medications 30 of each first housing 15 fall by gravity into corresponding second housings 19 of the second body 16, passing through the window 5, as shown in Fig. 12. In this case, the second body 16 is still in said initial position.

Once the medications 30 are fully held in the second housings 19, the second motor 28, through the toothed wheel 20 acting on the rack 21, axially moves the second body 16, until the first on the right second housing 19 reaches a first intermediate position wherein the first on the right second housing 19 is vertically aligned with the release opening 27 of the second casing 3. In this intermediate position of the second body 16, the particle/s 30 contained in the first on the right second housing 19 can fall down in the release mouth 26.

A control unit 11 controls the activation of first motor 25 and second motor 28 and consequently the rotation of first body 12 and the feed of second body 16. The control unit comprises a microprocessor and a program loaded into the microprocessor.

Fig. 13 shows the device 1 when the third second housing 19 on the right is vertically aligned with the release opening 27, but the skilled man can easily understand that the second body 16 intermittently moves between intermediate positions, as explained for the first intermediate position, until a final position is reached. The final position is the position in which the first on the left second housing 19, thus the last one, is vertically aligned with the release opening 27, as shown in Fig. 14.

In each intermediate position of the second body 16 a second housing 19 is aligned with the release opening 27 and consequently the article/s 30 contained in said second housing 19 drops outside the device 1 through the release mouth 26. Once the second body 16 has reached its final position, shown in Fig. 14, the control unit 11 acts the second motor 28 in such a way that the second body 16 is brought back to its initial position shown in Fig. 11.

In an embodiment (not shown) of the particle dispenser 1, the release opening 27, and the corresponding release mouth 26, can be arranged below the second housing 19 arranged at the end of the second body 16, in order to dispense particle/s 30 from this second housing 19 even when the second body 16 is in said initial position. This means that, when the first body 12 overturns for filling the second housings 19, one or more particles 30 are immediately released from the second housing arranged at the end of the second body 16.

Optionally, the first body 12 and/or the second body 16 comprise gaskets to guarantee the preservation of the medications contained in the particle dispenser 1.

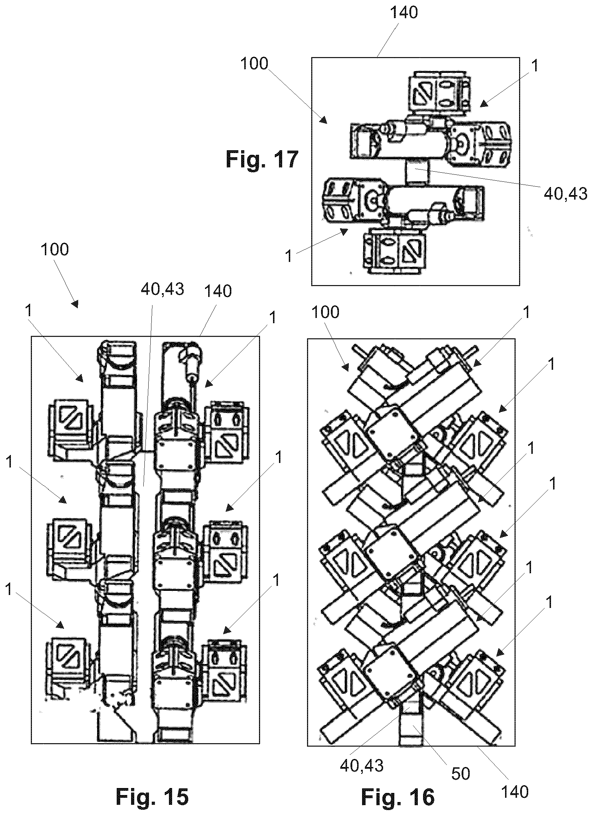

In Figs. 15-17 is shown a first embodiment of a dispensing system 100. A dispensing system 100 is a group of particle dispensers 1 functionally interconnect to each other. Each particle dispenser 1 conveys the articles/particles 30 in an inclined or vertical tube 43 that downwardly ends in a collecting tray 50. The inclined/vertical tube 43 acts as convey means 40, conveying the particles 30 in the collecting tray 50. The particles 30 expelled by the particle dispensers 1 fall by gravity into the collecting tray 50, from which the user can retrieve the particle 30 to be taken. In this embodiment of the dispensing system 100 even the particle dispensers 1 are arranged in an inclined manner within the outer casing 140, for reducing the footprint of the system 100. The embodiment of Figs. 15-17 has a vertical development for taking up less space on a table or for hugging it to a wall.

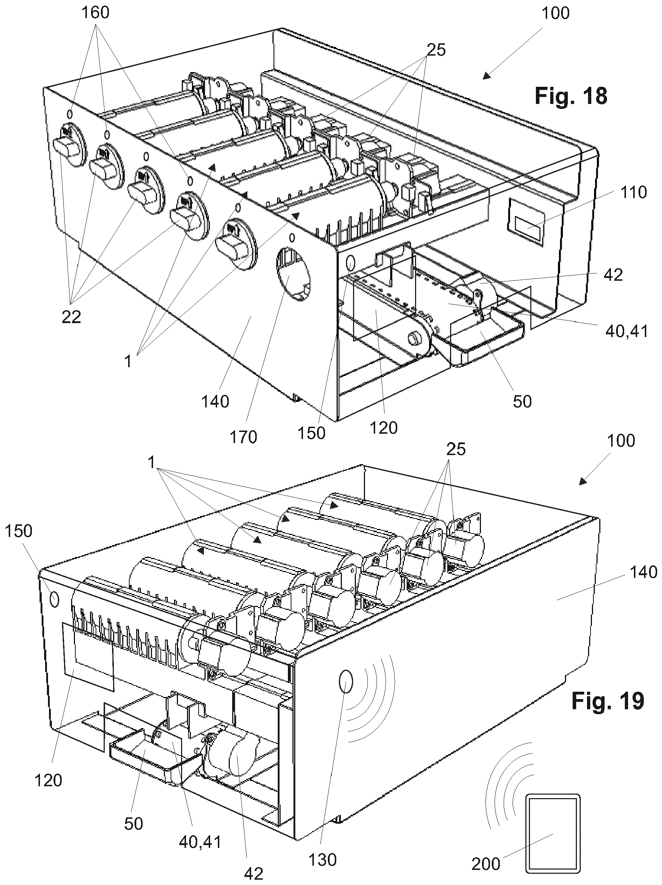

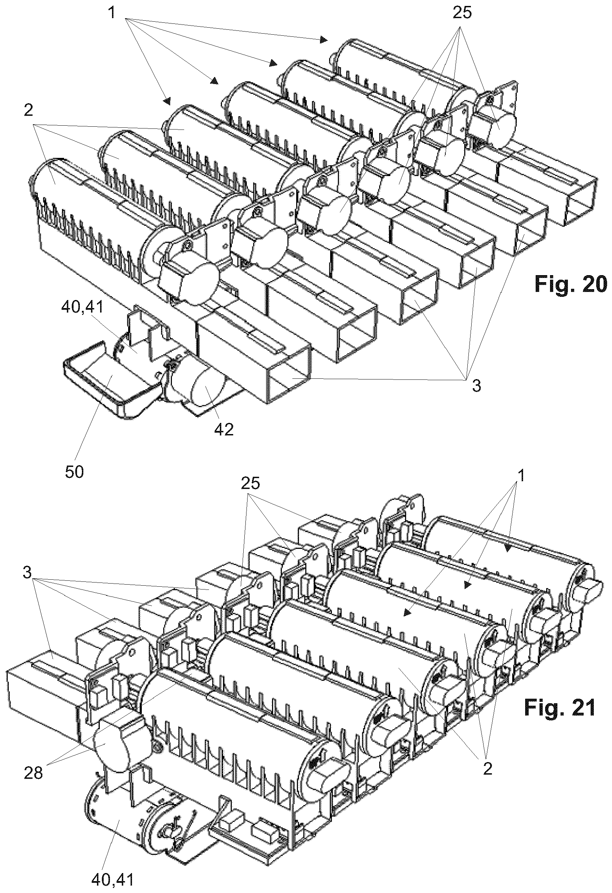

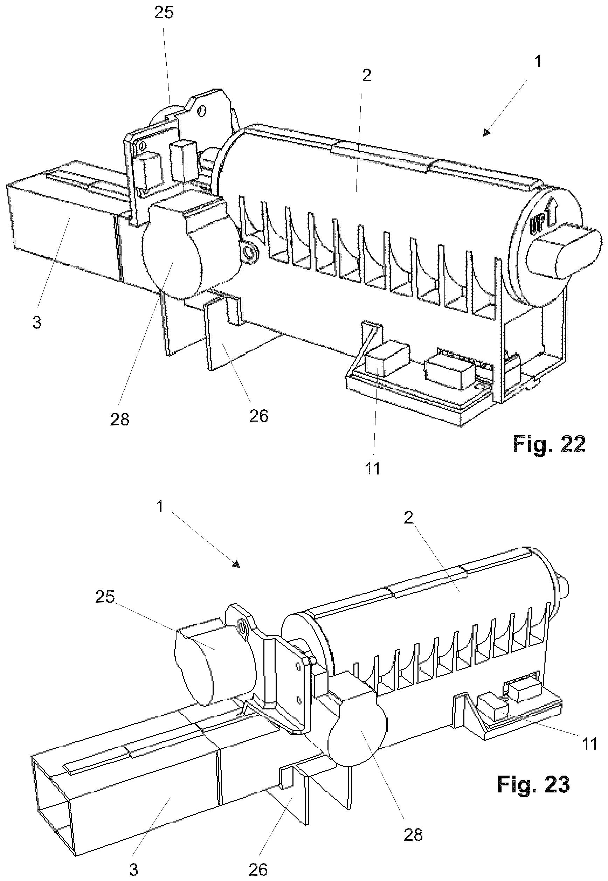

Figs. 18-21 show another embodiment of the dispensing system 100. In this embodiment of the dispensing system 100, the particle dispensers 1 are arranged parallel to each other and the ends of each first body 12 cantilever, on the same side, with their handles 22 from the outer casing 140. In this manner, the refilling of the first bodies 12 is easier. Each particle dispenser 1 comprises a release mouth 26, shown in detail in Figs. 22-25, which releases the particles 30 over a belt conveyor 41. The convey means 40 of this embodiment is the belt conveyor 41. The belt conveyor 40 conveys the particles 30 collected toward a collecting tray 50 in which the particles 30 are released.

Each time that at least a particle 30 is released on the belt conveyor 41, the belt conveyor 41 drags the particle 30 in the collecting tray 50.

The central control unit 110 of the dispensing system 100 is configured to command each particle dispenser 1 based on instructions received through a human-machine interface 120 and/or through a portable device 200, like a smartphone or tablet.

A user, a nurse or a doctor can refill the particle dispensers 1 with the medications 30 that a patient needs, and it can insert the dosage to follow for each medication through the human-machine interface 120 or the portable device 200. Once that the dispensing device 100 is set, the medications will be conveyed to the collecting tray 50 as prescribed in the posology.

The posology are converted by the central control unit 110 in instructional signals for commanding the motors 25, 28, 42 of the dispensing system 100. For example, the posology can prescribe one medication 30 from the first particle dispenser 1, which contains heart medications, and another medication 30 from the last particle dispenser 1, which contains diabetic drugs, each 8 hours. When an internal clock of the central control unit 110 registers that it has been 8 hours, the second motors 28 of the first and last particle dispensers 1 are commanded by the respective control units 11, thanks to instructional signals given by the central control unit 110, and the particles 30 prescribed are released on the belt conveyor 41, which in turn moves the medications 30 to the collecting tray 50.

The central control unit 110 is configured to control the control unit 11 of each particle dispenser 1. When the posology, inputted through the human-machine interface 120 or the mobile device 200, foresees particle/s 30 emission, at least one first motor 25 is activated and one or more first housings 15 are brought in correspondence of the respective release opening 27. Contemporary, a third motor 42 coupled to the belt conveyor 40 is commanded to perform a half turn, so that each particle 30 on the belt conveyor 40 reaches the collecting tray 50.

The dispensing system 100 also comprises a warning 150, that can be a warning light and/or speaker, for informing the user of the presence of one or more particles 30 in the collecting tray 50. Even the warning 150 is connected and controlled by the central control unit 110.

The mobile device 200 can comprise an internal memory configured to store a program adapted to manage the posology of particles/medications 30, a processor connected to said internal memory is adapted to execute said program and comprises communication means configured to transmit/receive data to/from a wireless communication system 130 belonging to the dispensing system 100. In turn, the wireless communication system 130 is connected to the central control unit 110. The wireless communication system 130 is of a known type, like a Bluetooth or wi-fi system. Through a wi-fi connection, the scheduling and posology can be managed remotely.

Even if it is not shown, the dispensing system 100 and the particle dispenser 1 are powered through a connection to the electric grid and all the motors 25, 28, 42 are electric as well as the control units 11, 110, the warning 150, the human-machine interface 120 and the wireless communication system 130.

Optionally, the collecting tray 50 can comprise a load cell (not shown) for detecting if the medication 30 has been collected from the collecting tray 50 or not. Alternative systems like a camera or IR detector can be used to check if the collecting tray 50 is empty or not.

The dispensing system 100 can also comprise an indicator light 160 which turns on when the second body 16 is in said final position, or in the intermediate position preceding said final position, in order to inform that corresponding particle dispenser is empty or will be emptied soon.

Alternatively, the indicator light 160 can turn on when the first body 12 assumes said first operating position. Indeed, this operating position corresponds to a discharge of particles 30 from the first body 12 into the second body 16. Once the first body 12 is empty, it can be extracted from the dispensing system 100 and refilled with new particles 30, while the second body 16 and the rest of the particle dispenser 1 continues to work. This feature allows a continuous dispensing of medications 30 without interruptions.

The dispensing system 100 of Fig. 18 misses a particle dispenser 1 for showing its modularity. The outer casing 140 can comprise one or more holes 170 for accommodating the charging end of the particle dispenser/s 1. If a specific dispensing system 100, like that of Fig. 19, needs all the slots occupied, to every hole 170 corresponds a particle dispenser 1. Otherwise, as Fig. 18 depicts, one or more holes 170 are empty and in place of particle dispenser a cork (not shown) can be arranged to close the hole/s 170.

Concluding, the invention so conceived is susceptible to many modifications and variations.

### Legend of reference signs:

1 particle dispenser
2 first casing
3 second casing
4 body (of first casing)
4A first end (of first casing)
4B second end (of first casing)
5 window
6 cylindrical bore (of first casing)
7 bore (of second casing)
8 support
9 bottom (of second casing)
10 sidewall (of second casing)
11 control unit
12 first body
13 semi-cylindrical sidewall (of first body)
14 partition (of first body)
15 first housing (of first body)
16 second body
17 sidewall (of second body)
18 partition (of second body)
19 second housing (of second body)
20 toothed wheel
21 rack
22 handle
23 connector
24 quick coupling
25 first motor (of first body)
26 release mouth
27 release opening
28 second motor (of second body)
30 particle/medication
40 convey means
41 belt conveyor
42 third motor
43 inclined/vertical tube
50 collecting tray
100 dispensing system
110 central control unit
120 human machine interface
130 wireless communication system
140 outer casing
150 warning
160 indicator light
170 hole (of the outer casing)
200 mobile device

## Claims

1. Particle dispenser (1) comprising:
- a casing (2,3);
- a first body (12) accommodated in said casing (2,3) and comprising a plurality of first housings (15) capable of containing a number of discrete particles (30);
- a second body (16) accommodated in said casing and comprising a plurality of second housings (19);
wherein the first body (12) is configured to move relative to said casing (2,3) between a first operating position in which each housing of the plurality of first housings (15) communicates with a corresponding housing of the second plurality of second housings (19) through a window (5) and a second operating position in which the first housings (15) are isolated from the second housings (19);
wherein the second body (16) is configured to selectively move relative to said casing (2,3) between an initial position to a final position passing through intermediate positions, wherein in each of said positions, included or excluded the initial position, at least one second housing (19) communicates with a release mouth (26) of the casing (2,3).

2. Particle dispenser (1) according to claim 1, wherein the casing (2,3) comprises a first casing (2) shaped for accommodating the first body (12) and a second casing (3) shaped for accommodating the second body (16).

3. Particle dispenser (1) according to claim 2, wherein first casing (2) defines a cylindrical bore (6) having a longitudinal axis, preferably said axis being arranged horizontal or substantially horizontal, and the first body is cylindrically shaped and is arranged coaxial to the axis of the hollow cylindrical bore (6) of said first casing (2).

4. Particle dispenser (1) according to claim 3, wherein the first body (12) comprises a cylindrical sidewall (13) that is partially opened along a longitudinal direction and a plurality of partitions (14) arranged so as to define said plurality of first housings (15), preferably said partitions (14) are perpendicular to a longitudinal axis of the first body (12).

5. Particle dispenser (1) according to claim 3, wherein the hollow cylindrical bore (6) of the first casing (2) is opened at one or both ends (4A,4B) and the first body (12) is selectively removable from the first casing (2) through one of its ends (4A) for refilling it.

6. Particle dispenser (1) according to any one of claims 3 to 5, wherein the second body (16) is prismatically coupled to the second casing (3), so that the second body (16) longitudinally moves along a direction that is substantially parallel to the longitudinal axis (X) of the first casing (2).

7. Particle dispenser (1) according to any one of preceding claims, wherein the second body (16) comprises a sidewall (17) having top and bottom apertures (11) and a plurality of partitions (18) arranged so as to define said plurality of second housings (19).

8. Particle dispenser (1) according to any one of preceding claims, wherein the first body (12) is moved by a first motor (25), preferably the first motor (25) is reversibly coupled to the first body (12) through a quick coupling (24), and the second body (16) is moved by a second motor (29).

9. Particle dispenser (1) according to any one pf preceding claims, further comprising a control unit (31) that is configured to control the second motor (29) so as to move forward the second body (16) from the initial position to each of the intermediate positions in a step by step manner until the final position is reached and backward from the final position to the initial position.

10. Particle dispenser (1) according to claim 9, wherein the control unit (31) moves forward the second body (16) from a position to the subsequent position after a predetermined time interval or if a command is provided to the control unit (31).

11. Particle dispenser (1) according to claim 9 or 10, wherein the control unit (31) is configured to move the first body (12) in the first operating position only when the second body (16) is positioned in said initial position.

12. Dispensing system (100) comprising:
- a plurality of particle dispensers (1) according to one or more of preceding claims;
- convey means (40) configured to collect and convey the particles (30) expelled from the particle dispensers (1) to a collecting tray (50).

13. Dispensing system (100) according to claim 12, wherein the convey means (40) comprise a belt conveyor (41) actuated by a third motor (42) or an inclined/vertical tube (43), ending in the collecting tray (50).

14. Dispensing system (100) according to claim 12 or 13, comprising a central control unit (110) which substitutes or coordinates the control units (31) of the particle dispensers (1) and a human-machine interface (120) and/or a wireless communication system (130) configured to communicate with a mobile device (200) of a user.

15. Dispensing system (100) according to claim 12, 13 or 14, comprising at least an indicator light (160) configured to turn on when the first body (12) of a particle dispenser (1) is empty and/or a warning (150) configured to switch-on when a particle (30) is contained in the collecting tray (50).

## Patentansprüche

1. Partikelspender (1) umfassend:
- ein Gehäuse (2,3);
- einen ersten Körper (12), der in dem Gehäuse (2,3) untergebracht ist und eine Vielzahl von ersten Gehäusen (15) umfasst, die eine Anzahl diskreter Partikel (30) enthalten können;
- einen zweiten Körper (16), der in dem Gehäuse untergebracht ist und eine Vielzahl von zweiten Gehäusen (19) umfasst;
wobei der erste Körper (12) so konfiguriert ist, dass er sich relativ zu dem Gehäuse (2, 3) zwischen einer ersten Betriebsposition bewegt, in der jedes Gehäuse der mehreren ersten Gehäuse (15) mit einem entsprechenden Gehäuse der zweiten Vielzahl zweiter Gehäuse kommuniziert (19) durch ein Fenster (5) und eine zweite Betriebsposition, in der die ersten Gehäuse (15) von den zweiten Gehäusen (19) isoliert sind;
wobei der zweite Körper (16) so konfiguriert ist, dass er sich relativ zu dem Gehäuse (2,3) selektiv zwischen einer Anfangsposition und einer Endposition bewegt und dabei Zwischenpositionen durchläuft, wobei in jeder dieser Positionen, einschließlich oder außerhalb der Anfangsposition, mindestens ein zweites Gehäuse (19) mit einer Auslöseöffnung (26) des Gehäuses (2,3) kommuniziert.

2. Partikelspender (1) nach Anspruch 1, wobei das Gehäuse (2,3) ein erstes Gehäuse (2) umfasst, das so geformt ist, dass es den ersten Körper (12) aufnehmen kann, und ein zweites Gehäuse (3), das so geformt ist, dass es den zweiten Körper (16) aufnehmen kann.

3. Partikelspender (1) nach Anspruch 2, bei dem das erste Gehäuse (2) eine zylindrische Bohrung (6) mit einer Längsachse definiert, wobei die Achse vorzugsweise horizontal oder im Wesentlichen horizontal angeordnet ist, und der erste Körper eine zylindrische aufweist und koaxial zur Achse der hohlzylindrischen Bohrung (6) des ersten Gehäuses (2) angeordnet ist.

4. Partikelspender (1) nach Anspruch 3, wobei der erste Körper (12) eine zylindrische Seitenwand (13) umfasst, die teilweise entlang einer Längsrichtung geöffnet ist, und eine Vielzahl von Trennwänden (14), die so angeordnet sind, dass sie die Vielzahl von ersten Gehäusen (15) definieren, wobei die Trennwände (14) vorzugsweise senkrecht zu einer Längsachse des ersten Körpers (12) verlaufen.

5. Partikelspender (1) nach Anspruch 3, wobei die hohle zylindrische Bohrung (6) des ersten Gehäuses (2) an einem oder beiden Enden (4A, 4B) geöffnet ist und der erste Körper (12) zum Nachfüllen selektiv durch eines seiner Enden (4A) aus dem ersten Gehäuse (2) entfernbar ist.

6. Partikelspender (1) nach einem der Ansprüche 3 bis 5, wobei der zweite Körper (16) prismatisch mit dem zweiten Gehäuse (3) gekoppelt ist, so dass sich der zweite Körper (16) in Längsrichtung entlang einer Richtung bewegt, die im Wesentlichen parallel zur Längsachse (X) des ersten Gehäuses (2) ist.

7. Partikelspender (1) nach einem der vorhergehenden Ansprüche, wobei der zweite Körper (16) eine Seitenwand (17) mit oberen und unteren Öffnungen (11) und eine Vielzahl von Trennwänden (18) umfasst, die so angeordnet sind, dass sie die Vielzahl von zweiten Gehäusen (19) definieren.

8. Partikelspender (1) nach einem der vorhergehenden Ansprüche, wobei der erste Körper (12) durch einen ersten Motor (25) bewegt wird, vorzugsweise ist der erste Motor (25) reversibel mit dem ersten Körper (12) gekoppelt. über einen Schnellverbinder (24) und der zweite Körper (16) wird durch einen zweiten Motor (29) bewegt.

9. Partikelspender (1) nach einem der vorhergehenden Ansprüche, weiterhin umfassend eine Steuereinheit (31), die dazu konfiguriert ist, den zweiten Motor (29) so zu steuern, dass er den zweiten Körper (16) von dem nach vorne bewegt Von der Ausgangsposition aus schrittweise zu jeder Zwischenposition, bis die Endposition erreicht ist, und von der Endposition zurück zur Ausgangsposition.

10. Partikelspender (1) nach Anspruch 9, wobei die Steuereinheit (31) den zweiten Körper (16), nach einem vorgegebenen Zeitintervall, oder wenn der Steuereinheit (31) ein Befehl bereitgestellt wird, von einer Position zur nächsten Position vorwärts bewegt.

11. Partikelspender (1) nach Anspruch 9 oder 10, wobei die Steuereinheit (31) so konfiguriert ist, dass sie den ersten Körper (12) nur dann in die erste Betriebsposition bewegt, wenn der zweite Körper (16) in der Anfangsposition positioniert ist.

12. Abgabesystem (100) umfassend:
- eine Vielzahl von Partikelspendern (1) nach einem oder mehreren der vorhergehenden Ansprüche;
- Fördermittel (40), die dazu ausgebildet sind, die aus den Partikelspendern (1) ausgestoßenen Partikel (30) zu sammeln und zu einer Auffangschale (50) zu befördern.

13. Abgabesystem (100) nach Anspruch 12, wobei die Fördermittel (40) ein von einem dritten Motor (42) angetriebenes Förderband (41) oder ein geneigtes/vertikales Rohr (43) umfassen, das in der Auffangschale (50) endet.

14. Abgabesystem (100) nach Anspruch 12 oder 13, umfassend eine zentrale Steuereinheit (110), die die Steuereinheiten (31) der Partikelspender (1) ersetzt oder koordiniert, und eine Mensch-Maschine-Schnittstelle (120) und/oder ein drahtloses Kommunikationssystem (130), das dazu ausgebildet ist, mit einem mobilen Gerät (200) eines Benutzers zu kommunizieren.

15. Abgabesystem (100) nach Anspruch 12, 13 oder 14, das mindestens eine Kontrollleuchte (160) umfasst, die sich einschaltet, wenn der erste Körper (12) eines Partikelspenders (1) leer ist, und/oder eine Warnung (150), die sich einschaltet, wenn sich ein Partikel (30) in der Auffangschale (50) befindet.

## Revendications

1. Distributeur de particules (1) comprenant :
- un boîtier (2,3) ;
- un premier corps (12) logé dans ledit boîtier (2,3) et comprenant une pluralité de premiers logements (15) pouvant contenir un certain nombre de particules discrètes (30) ;
- un second corps (16) logé dans ledit boîtier et comprenant une pluralité de seconds logements (19) ;
dans lequel le premier corps (12) est configuré pour se déplacer par rapport audit boîtier (2,3) entre une première position de fonctionnement dans laquelle chaque logement de la pluralité de premiers logements (15) communique avec un logement correspondant de la seconde pluralité de seconds logements (19) à travers une fenêtre (5) et une seconde position de fonctionnement dans laquelle les premiers logements (15) sont isolés des seconds logements (19) ;
dans lequel le second corps (16) est configuré pour se déplacer sélectivement par rapport audit boîtier (2,3) entre une position initiale et une position finale en passant par des positions intermédiaires, dans lequel dans chacune desdites positions, y compris ou non la position initiale, au moins un second boîtier (19) communique avec une embouchure de libération (26) du boîtier (2,3).

2. Distributeur de particules (1) selon la revendication 1, dans lequel le boîtier (2,3) comprend un premier boîtier (2) formé pour recevoir le premier corps (12) et un second boîtier (3) formé pour recevoir le second corps (16).

3. Distributeur de particules (1) selon la revendication 2, dans lequel le premier boîtier (2) définit un alésage cylindrique (6) ayant un axe longitudinal, de préférence ledit axe étant disposé horizontalement ou sensiblement horizontal, et le premier corps est de forme cylindrique et est disposé coaxialement à l'axe de l'alésage cylindrique creux (6) dudit premier boîtier (2).

4. Distributeur de particules (1) selon la revendication 3, dans lequel le premier corps (12) comprend une paroi latérale cylindrique (13) partiellement ouverte selon une direction longitudinale et une pluralité de cloisons (14) disposées de manière à définir ladite pluralité de premiers logements (15), de préférence lesdites cloisons (14) sont perpendiculaires à un axe longitudinal du premier corps (12).

5. Distributeur de particules (1) selon la revendication 3, dans lequel l'alésage cylindrique creux (6) du premier boîtier (2) est ouvert à une ou aux deux extrémités (4A, 4B) et le premier corps (12) peut être retiré sélectivement du premier boîtier (2) par l'une de ses extrémités (4A) pour le remplir à nouveau.

6. Distributeur de particules (1) selon l'une quelconque des revendications 3 à 5, dans lequel le second corps (16) est couplé de manière prismatique au second boîtier (3), de sorte que le second corps (16) se déplace longitudinalement le long d'une direction qui est sensiblement parallèle à l'axe longitudinal (X) du premier boîtier (2).

7. Distributeur de particules (1) selon l'une quelconque des revendications précédentes, dans lequel le second corps (16) comprend une paroi latérale (17) ayant des ouvertures supérieure et inférieure (11) et une pluralité de cloisons (18) agencées de manière à définir ladite pluralité de seconds logements (19).

8. Distributeur de particules (1) selon l'une quelconque des revendications précédentes, dans lequel le premier corps (12) est déplacé par un premier moteur (25), de préférence le premier moteur (25) est couplé de manière réversible au premier corps (12) par l'intermédiaire d'un raccord rapide (24), et le second corps (16) est déplacé par un second moteur (29).

9. Distributeur de particules (1) selon l'une quelconque des revendications précédentes, comprenant en outre une unité de commande (31) qui est configurée pour commander le second moteur (29) de manière à déplacer le second corps (16) vers l'avant de la position initiale à chacune des positions intermédiaires de manière pas à pas jusqu'à ce que la position finale soit atteinte et vers l'arrière de la position finale à la position initiale.

10. Distributeur de particules (1) selon la revendication 9, dans lequel l'unité de commande (31) déplace le second corps (16) vers l'avant d'une position à la position suivante après un intervalle de temps prédéterminé ou si une commande est fournie à l'unité de commande (31).

11. Distributeur de particules (1) selon la revendication 9 ou 10, dans lequel l'unité de commande (31) est configurée pour déplacer le premier corps (12) dans la première position de fonctionnement uniquement lorsque le second corps (16) est positionné dans ladite position initiale.

12. Système de distribution (100) comprenant :
- une pluralité de distributeurs de particules (1) selon une ou plusieurs des revendications précédentes ;
- des moyens de convoyage (40) configurés pour collecter et convoyer les particules (30) expulsées des distributeurs de particules (1) vers un bac de récupération (50).

13. Système de distribution (100) selon la revendication 12, dans lequel les moyens de convoyage (40) comprennent un convoyeur à bande (41) actionné par un troisième moteur (42) ou un tube incliné/vertical (43), aboutissant au bac de récupération (50).

14. Système de distribution (100) selon la revendication 12 ou 13, comprenant une unité de commande centrale (110) qui se substitue ou coordonne les unités de commande (31) des distributeurs de particules (1) et une interface homme-machine (120) et/ou un système de communication sans fil (130) configuré pour communiquer avec un dispositif mobile (200) d'un utilisateur.

15. Système de distribution (100) selon la revendication 12, 13 ou 14, comprenant au moins un voyant lumineux (160) configuré pour s'allumer lorsque le premier corps (12) d'un distributeur de particules (1) est vide et/ou un avertisseur (150) configuré pour s'allumer lorsqu'une particule (30) est contenue dans le bac de récupération (50).
